# EUROPEAN PATENT APPLICATION

(11) **EP 4 431 159 A1**
(43) Date of publication of application: **18.09.2024**
(21) Application number: 24162256.2
(22) Date of filing: 08.03.2024
(51) Int. Cl.: A61Q 11/00, A61K 8/73, A61K 8/86, A61C 1/00, A61C 17/18

(54) **COMPOSITION FOR REDUCING OR ELIMINATION AEROSOL GENERATION DURING DENTAL AND SURGICAL PROCEDURES**

(30) Priority: 13.03.2023 EP 23161637
(71) Applicant: Ivoclar Vivadent AG, 9494 Schaan (LI)
(72) Inventor: Schädlich, Johannes, 9430 St. Margethen (CH); Köhler, Thomas, 78479 Reichenau (DE)
(74) Representative: Uexküll & Stolberg

(57) **Abstract**

A composition for reducing/eliminating aerosolization during dental/surgical irrigation procedures comprising at least one liquid or solid carrier and at least one polymer, wherein the at least one polymer comprises at least hydroxyalkyl cellulose polymer, at least one polyethylene oxide) polymer or a mixture thereof and wherein the at least one polymer is not soluble in the liquid carrier.

## Description

Dental procedures or surgical procedures involving rotary, laser, kinetic, and ultrasonic instruments utilize water irrigation that becomes aerosolized during the procedure. Aerosols (10⁻⁴ to 10 µm) are known to carry viruses (*e.g*., SARS, SARS-CoV-2). Aerosols and droplets generated can be carried for long distances during these procedures. Specifically, during the performance of certain dental/surgical procedures, aerosolization can arise from the use of water in the procedures. With the potential of aerosol droplets carrying viruses or other microbes, extensive time and cost may need to be expended for infection management and as such, strategies are needed for safely carrying out dental and other surgical procedures, particularly involving patients carrying contagious viruses, such as SARS-CoV-2.

Furthermore, the generation of droplets and aerosols during handling of dental rotary, kinetic and ultrasonic equipment may reduce visibility of the working field for the operator, especially when using magnifying loupes. Aerosols and droplets also lead to misting of glasses, goggles and face shields and contaminate the patient's body and clothing and nearby working surfaces.

US 7,566,448 B2 discloses reduced aerosol generating personal care and cleaning products comprising high molecular weight polyethylene oxide, an enzyme, an enzyme protecting agent and one or more personal care or cleaning product components. The high molecular weight polyethylene oxide serves as an anti-misting agent to reduce the potential of aerosol generation and preferably has a molecular weight of 400,000 to 7 million g/mol.

Plog et al. (Phys. Fluids 32, 083111, 2020) found that the formation of aerosol particles by rotary and ultrasonic instruments used in medicine and dentistry and the dispersion of aerosols beyond the point of origin can be reduced or completely eliminated by the addition of polyacrylic acid or xanthan gum to the water used by the instruments.

WO 2021/242930 discloses the use of one or more polymers for reducing or eliminating formation of aerosol droplets during a medical or dental procedure. The polymers are used in the form of an aqueous composition having a shear viscosity of 10⁻³-10³ poise and an elongational viscosity of 10² to 10⁷ poise. The polymers are chosen from poly(ethylene oxide), xanthan gum, poly(vinyl pyrrolidone), alginic acid, hyaluronic acid, chondroitin sulfate, and combinations thereof.

According to the prior art, polymers are dissolved in water to reduce aerosol formation during dental treatments. Organic polymers and in particular high molecular weight polymers are difficult to dissolve in water and require a long mixing time, making them difficult to use. On the other hand, it was found that aqueous polyethylene oxide) solutions lose their ability to suppress aerosol formation on storage. The reason for this is not yet known. Due to the lower storage stability, it is not desirable to deliver aqueous solutions to the end user.

It is an object of the present invention to provide compositions which are suitable for reducing or elimination aerosol generation during dental and surgical procedures which do not have the disadvantages of the prior art. In particular, it is an object to provide compositions which are stable and can be easily handled by the end user, such as a dentist.

According to the present invention, this object is achieved by compositions comprising at least one liquid or solid carrier and at least one polymer. The at least one polymer is preferably a hydroxyalkyl cellulose polymer, at least one polyethylene oxide) polymer or a mixture thereof, more preferably a poly(ethylene oxide) polymer. The at least one polymer is preferably solid at room temperature and water soluble. The polymer preferably has an average particle size of 20 to 2000 µm.

The at least one hydroxyalkyl cellulose polymer is preferably hydroxypropyl cellulose and more preferably hydroxyethyl cellulose. The hydroxyalkyl cellulose polymer has preferably an average molecular weight of 300,000 to 450,000 g/mol, more preferably 370,000 to 380,000 g/mol and most preferably about 380,000 g/mol.

The at least one polyethylene oxide) polymer has preferably an average molecular weight of 600,000 to 12 million g/mol, more preferably 3 million to 8 million g/mol, even more preferably 7 million to 8 million g/mol and most preferably about 8 million g/mol. In the following high molecular weight poly(ethylene oxides) are also referred to as PEO. The poly(ethylene oxide) polymers may be branched or straight chain polymers. Straight chain polymers are preferred. The PEO preferably has an average particle size of 20 to 2000 µm.

The compositions according to the present invention may comprise a solid carrier or preferably a liquid carrier. Preferred liquid carriers are biocompatible liquids, in particular liquids in which the polymer is not soluble. Liquid carriers are compounds which are liquid at room temperature (22°C) and preferably at 0°C. In the context of the invention, liquids in which the polymer is not soluble are liquids that dissolve no more than 30 wt.%, preferably no more than 20 wt.%, more preferably no more than 10 wt.% and most preferably no more than 5 wt.% of the polymer present in the composition at room temperature. The liquid carrier is preferably water miscible.

Preferred liquid carriers are alcohols, more preferably polyols, fatty acids, ethers, such as tetrahydrofuran, and DMSO. Preferred polyols are ethylene glycol, propylene glycol, glycerol, low molecular weight polyethylene oxides) and mixtures thereof. Preferred low molecular weight poly(ethylene oxides) are poly(ethylene oxides) with an average molecular weight of 200 to 600 g/mol, preferably 400 g/mol. In the following low molecular weight poly(ethylene oxides) will also be referred to as polyethylene glycols (PEG). Preferred alcohols with one hydroxyl group are ethanol, propane-1-ol, propan-2-ol, and fatty alcohols with 6 to 10 carbon atoms. Particularly preferred liquid carriers are glycerol, PEG 400, i.e. polyethylene glycol with an average molecular weight of 400 g/mol, and mixtures thereof.

The at least one polyethylene oxide) with an average molecular weight of 200 to 600 g/mol and the at least one poly(ethylene oxide) with a molecular weight of 600,000 to 12 million g/mol are preferably used in a weight ratio of 1:2 to 1:3, preferably 1:2.6.

Preferred solid carriers are water soluble sugars, such as monosaccharides, preferably glucose, disaccharides, preferably sucrose, polysaccharides, sugar alcohols, preferably xylitol or sorbitol, citric acid, tartaric acid, solid low molecular weight polyethylene glycols, preferably PEG 1000, gelatin, polyacrylates, polyvinyl alcohols, gum arabic, saccharin, and cyclamate.

Further preferred solid carriers are water soluble salts, such as (NH₄)₂SO₄, K₂SO₄, Na₂SO₄, MgSO₄, NaCl, NaBr, MgCl₂, Na₂HPO₄, CaHPO₄, Na₂CO₃ and in particular NaHCO₃. Basic salts can be combined with a solid organic acid, such as tartaric acid or preferably citric acid. PEO, basic solid carrier and the solid acid can be compressed into tablets which dissolve rapidly in water with the generation of CO₂.

According to an alternative embodiment of the present invention, the hydroxyalkyl cellulose or preferably the high molecular polyethylene oxide) polymer is used in the form of a solid powder (e.g., granule or other form) that is used in combination with a powdery solid carrier, preferably NaHCO₃.

It is also possible to coat the PEO or hydroxyalkyl cellulose on the solid carrier, e.g., by dissolving the polymer in water and applying the polymer solution on a particulate solid carrier, preferably in a fluid bed dryer.

The solid carrier preferably has an average particle size of 20 to 2000 µm.

Compositions with a liquid carrier preferably comprise:
- 50 - 99 wt.%, preferably 69 - 97 wt.% and more preferably 90 wt.% of the liquid carrier, preferably glycerol and/or PEG 400; and
- 1 - 50 wt.%, preferably 3 - 31 wt.% and more preferably 10 wt.% of at least one hydroxyalkyl cellulose, preferably at least one poly(ethylene oxide) having a molecular weight of 600,000 to 12 million g/mol,
in each case based on the total mass of the composition.

Particularly preferred are compositions comprising:
- 36 - 71 wt.%, preferably 50 - 70 wt.% and more preferably 65 wt.% glycerol;
- 14 - 28 wt.%, preferably 19 - 27 wt.% and more preferably 25 wt.% of at least one at least one poly(ethylene oxide) having a molecular weight of 200 to 600 g/mol, and
- 1 - 50 wt.%, preferably 3 - 31 wt.% and more preferably 10 wt.% of at least one polyethylene oxide) having a molecular weight of 600,000 to 12 million g/mol,
in each case based on the total mass of the composition.

Further particularly preferred are compositions comprising:
- 36 - 71 wt.%, preferably 40 - 70 wt.% and more preferably 65 wt.% glycerol;
- 14 - 35 wt.%, preferably 19 - 35 wt.% and more preferably 25 wt.% of at least one at least one poly(ethylene oxide) having a molecular weight of 200 to 600 g/mol, and
- 1 - 29 wt.%, preferably 3-25 wt.% and more preferably 10 wt.% of at least one polyethylene oxide) having a molecular weight of 600,000 to 12 million g/mol,
in each case based on the total mass of the composition.

Compositions with a solid carrier preferably comprise:
- 1 to 80 wt.%, preferably 9 to 50 wt.%, of at least one hydroxyalkyl cellulose or preferably at least one poly(ethylene oxide) with a molecular weight of 600,000 to 12 million g/mol, preferably 8 million g/mol, and
- 20 to 99 wt.%, preferably 50 to 91 wt.%, of a solid carrier, preferably NaHCOs,
in each case based on the total mass of the composition.

Particularly preferred are compositions comprising:
- about 50 wt.% of at least one polyethylene oxide) with a molecular weight of 600,000 to 12 million g/mol, preferably 8 million g/mol, and
- about 50 wt.% of a solid carrier, preferably NaHCOs,
in each case based on the total mass of the composition.

The compositions may comprise various additives. Examples of additives include, but are not limited to antioxidants or antioxidant-synergists, chelating agents, flavorings, colorings, antimicrobials, preservatives, biofilm removal agents, calculus or plaque staining dyes, wetting agents/detergents, and the like, and combinations thereof. Preferred additives are thickeners. Preferred thickeners are precipitated and fumed silica. Particularly preferred is fumed silica having a specific BET surface area of 175 to 225 m²/g and an average particle size of 5 to 50 nm such as Aerosil 200. If present, the one or more additives are preferably used in a total amount of 0 to 5 wt.%, more preferably from 0 to 3.0 wt.%, and most preferably 0 to 2.0 wt.%.

In various embodiments, the composition further comprises one or more stabilizers. Non-limiting examples of stabilizers include those in the following table.

**Table 1. Non-limiting list of stabilizers.**

| **Name** | **CAS** | **Stabilizer type** | **Concentration** | **Preferred concentration** |
|---|---|---|---|---|
| BHT | 128-37-0 | antioxidants | 0.001-0.5% | 0.01-0.2% |
| BHA | 25013-16-5 | antioxidants | 0.001-0.5% | 0.01-0.2% |
| Ascorbic acid | 50-81-7 | antioxidants | 0.001-0.5% | 0.01-0.2% |
| Glutathion | 70-18-8 | antioxidants | 0.001-0.5% | 0.01-0.2% |
| EDTA | 6381-92-6 | Chelating agent | 0.001-0.5% | 0.01-0.2% |

Preferred preservatives are parabens, benzalkonium chloride, and benzyl alcohol.

Both the compositions with a liquid carrier and the compositions with a solid carrier preferably comprise no added water. They may comprise trace amounts of water present in the materials used to prepare the compositions. Preferably, the total amount of water in the compositions is less than 1.0 wt.%, more preferably less than 0.5 wt.% and most preferably less than 0.3 wt.%.

Thus, particularly preferred compositions with a liquid carrier comprise:
- 50 - 99 wt.%, preferably 69 - 97 wt.% and more preferably 90 wt.% of the liquid carrier, preferably glycerol and/or PEG 400; and
- 1 - 50 wt.%, preferably 3 - 31 wt.% and more preferably 10 wt.% of at least one hydroxyalkyl cellulose, preferably at least one poly(ethylene oxide) having a molecular weight of 600,000 to 12 million g/mol,
- 0 - 5 wt.%, preferably 0 - 3.0 wt.% and more preferably 0 - 2.0 wt.% of one or more additives,
- 0 - 0.5 wt.%, preferably 0.001 - 0.5% and more preferably 0.01 - 0.2 wt.% of one or more stabilizers, and
- 0 - 1.0 wt.%, preferably 0 - 0.5 wt.% and most preferably 0 - 0.3 wt.% of water,
in each case based on the total mass of the composition.

According to the present invention, compositions are preferred in which the amounts of the liquid or solid carrier, the at least one polymer, additives, stabilizers, and water add up to 100 wt.%.

For use, the compositions defined above are preferably mixed with water. Surprisingly, it was found that compositions comprising a high molecular weight polyethylene oxide) in combination with a liquid or solid carrier can be dispersed/dissolved in water much more easily than compositions without a carrier. Compositions that do not contain water are also referred to as concentrates in the following. It was also surprisingly found that the concentrates are stable during storage, i.e. do not lose their property of preventing the formation of aerosols.

The aqueous compositions obtained by mixing the liquid or solid concentrate with water are suitable for irrigation during dental or surgical procedures, where the treatment area is frequently irrigated with aqueous compositions. Also provided are systems and methods using the compositions. The compositions, systems and methods are particularly useful where there may be a potential risk of virucidal and microbial transfer, such as viral or bacterial transfer. The aqueous compositions of the present invention reduce aerosolization and avoid inconvenience to dentists and patients during dental and surgical operations.

In one aspect, the present invention provides a system for irrigation of a treatment area. For example, the system may comprise waterlines for use in a dental unit configured to deliver an aqueous composition comprising, consisting essentially of or consisting of one or more polymers. The compositions may be released into the dental treatment area under pressures routinely used during dental procedures. In various embodiments, a system may comprise a dental unit water bottle (also referred to herein as reservoir) comprising aqueous compositions comprising polymers, and waterlines for delivery of the composition to the dental treatment area. The system may further comprise a control means, such as a dental handpiece, for controlling the release or the flow of the composition into the dental treatment area.

The composition and systems may be used in surgical/dental procedures for irrigation of treatment areas. For example, the compositions may be used where there is likelihood of generation of aerosols due to irrigation with water under rotary, ultrasonic or kinetic agitation. Reduction or elimination of aerosols at the point of generation will aid in preventing the airborne/aerosol transmission of viruses. The use of unusual and extensive protective measures which require permanent or mobile installation in the operatory, increasing cost and time needed for surgical or dental procedures can be avoided. Using the methods, compositions, and systems of the present invention will aid in reducing the risk of aerosol disease transmission to patients, health care workers and by-standers. In addition, the reduction of aerosols during dental treatment improves field of visibility for the operator, allowing more precise and timely operation. The reduced aerosol and droplet formation is also beneficial to the patient by preventing contamination of exposed body parts or clothing with airborne residues from dental procedures.

Dentistry uses rotary, ultrasonic, kinetic, and laser-based instruments in the daily, routine treatment of patients. All of these require water irrigation or the use of air/water syringes to cool the tooth surface and/or to wash away debris from the tooth. In providing water irrigation, abundant and continuous aerosols are generated from the high pressure differences involved or by the high velocities of moving parts. These aerosols may extend for many feet beyond the patient's mouth and the area where the dentist is working. Multiple viruses, including the SARS-CoV-2 virus, and the related COVID-19 pandemic have spread throughout the United States and the rest of the world. Several investigations have demonstrated the SARS-CoV-2 virus is spread by both droplet (visible drops) and aerosol transmission. The generation of aerosols in dentistry - an unavoidable part of most dental/surgical facial cosmetic and irrigation treatments - creates a high-risk situation by potentially spreading the virus long distances to other people.

Compared to other administrative and engineering methods, the advantages of the compositions herein include one or more of: 1) direct mitigation of aerosol formation, 2) marked or complete aerosol elimination that is not dependent on human behavior, other than its inclusion in procedures, 3) low cost compared to engineering controls involving airflow (HVAC) or room construction, 4) simplicity as the irrigation solution replaces water used otherwise, 5) no additional hardware requiring sterilization between patients, 6) easily stored at room temperature, 7) widely adapted to most if not all clinical settings including those supporting poor access to care communities, 8) low tech = no training, 9) scalable from small to large organizations, 10) can be flavored/colored, and 11) does not require continuous human engagement.

A further expansion of the principles herein is for reduction of aerosol in surgical irrigation solution (typically 0.9% saline) to achieve the same reduction of aerosol in other surgical fields using water-based irrigation (for example orthopedic surgery or wound debridement). The irrigation solution reduces or completely eliminates aerosol generation. It is not anti-viral / bacterial nor intended to kill viruses/bacteria. Thus, clinical dentistry will still be required to use personal protective equipment (PPE) to protect workers natural aerosolization (speaking, coughing, sneezing).

In another aspect, the present disclosure provides compositions for use in medical or dental or surgical procedures that reduce aerosolization. The compositions comprise, preferably consists essentially of, or most preferably consists of the compounds specified above. The compositions do not take the form of a personal care or cleaning product. The composition according to the invention may further comprise one or more additives.

The compositions reduce formation of or eliminate aerosolized droplets. The term "reduction in formation of aerosolized droplets" or similar terms as used herein refers to a decrease of the number of aerosolized droplets produced during irrigation and/or mechanical agitation (such as, for example, during providing water irrigation to teeth, such as, for example, during dental procedures) of a liquid (e.g., water). For example, the amount of droplets generated during a dental cleaning procedure may be reduced. Reduction in aerosolization may result in suppression of droplet formation, including complete suppression of aerosol formation (*e.g*., elimination), in particular the formation of droplets having a size (diameter) of 0.1 µm to 50 µm. The aerosolized droplets may comprise the composition, water, and/or saliva.

If not stated otherwise, the average molecular weight of all polymers given herein is the weight average molecular weight Mw. Mw is preferably determined by gel permeation chromatography (GPC). Gel permeation chromatography (GPC) is a relative method in which the molecules are separated on the basis of their size, or more precisely on the basis of their hydrodynamic volume. The absolute molecular weight is determined by calibration with known standards. Preferably, narrowly distributed polystyrene standards are used as calibration standards. These are commercially available. Styrene-divinylbenzene columns are used as separation media and tetrahydrofuran (THF) as eluent. Styrene-divinylbenzene columns are suitable for organic soluble synthetic polymers. The measurement is carried out with dilute solutions (0.05 - 0.2 wt.%) of the polymers to be investigated.

Alternatively, the weight average molecular weight can be determined by the known methods of freezing point depression (cryoscopy), boiling point elevation (ebullioscopy) or from vapor pressure depression (vapor pressure osmometry). These are absolute methods that do not require calibration standards. Concentration series of 4 to 6 diluted polymer solutions with concentrations of 0.005 to 0.10 mol/kg are examined and then the measured values are extrapolated to a concentration of 0 mol/kg.

Unless otherwise stated, all particle sizes are weight-average particle sizes, wherein particle-sizes within the range of from 0.1 µm to 2000 µm are measured by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared, and the scattered light thereof is measured in a flow cell. The scattered-light analysis for calculating particle size and particle-size distribution is performed in accordance with the Mie theory according to DIN/ISO 13320: 2020.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably with a Malvern Zetasizer Nano ZS (Malvern Instruments, Malvern UK) with a He-Ne laser with a wavelength of 633 nm, at a scattering angle of 90° at 25° C.

Particle sizes smaller than 0.1 µm can also be determined by means of SEM or TEM spectroscopy. The transmission electron microscopy (TEM) is preferably carried out with a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

Advantages of the present compositions include that they do not affect downstream events, such as dental procedures, its components (such as, for example, PEO) can be washed away quickly and therefore, do not linger in the treatment area. It is may be vital for subsequently performed dental procedures that there be no detrimental effect (*e.g*., on bond adhesion etc.) caused by the present composition.

In a further aspect, this disclosure provides a dental unit system comprising a dental unit bottle and waterline configured to deliver a composition comprising an aqueous polymer composition as described herein to a dental treatment area in an individual, such that the generation of aerosol particles upon release of the composition into the dental treatment area during dental procedures is reduced (*e.g*., relative to water alone). The system may also optionally comprise a hand-piece for controlling the release of the composition into the dental treatment area. The system may be configured to use a premade ready-to-use aerosolization reducing composition or may be configured to dilute a concentrated aerosolization reducing composition in transit at any point from the reservoir containing the concentrated composition to the point of use in the treatment area. This may be done for dental appliances with a water tank (e.g. mobile dental scaling units) by replacing the commonly used water in the tank with an embodiment of the present composition. On the other hand, non-mobile dental appliances (*e.g*., stationary ultrasonic scalers) can be built into the dental chair and attached to the main water supply (dental unit waterline). Such appliances may have a port to continuously introduce disinfectants that prevent biofouling of the liquid feed system. This port may be used to feed the correct amount of a concentrate of present composition into the water line that is diluted to desired concentrations during its passage through the liquid feed system to the handpiece.

In a further aspect, the present disclosure may be embodied as a system having low aerosolization in a dental instrument (*e.g*., a water syringe, air/water syringe, ultrasonic scaler, etc.) A system may have a reservoir containing a composition according to the present invention. A supply line is in fluid communication with the reservoir. The supply line is configured to supply the composition of the reservoir to the dental distribution as an aqueous composition at a final concentration (*i.e*., the concentration of the aqueous composition delivered to the instrument). The final concentration of the high molecular weight polyethylene oxide) is preferably within a range to 100 to 1000 ppm, relative to the total weight of the aqueous composition. Embodiments of the system may be configured for a final concentration according to any of the compositions disclosed herein.

In some embodiments, the composition of the invention is added into the fluid of the supply line during flow of the fluid. For example, the compositions may be drawn into the fluid flow using the Venturi effect. In other embodiments, the reservoir may be pressurized such that the composition is injected into the supply line (*e.g*., automatically, selectively, etc.) In such embodiments, the composition may be delivered to the supply line as a fluid.

In such embodiments, the flow of fluid from the supply line (or a portion of the flow) may be directed into the reservoir to mix with/dissolve the powder mixture for subsequent (downstream) use with a patient. Such an embodiment may also be used with a liquid composition.

In some embodiments, combinations of techniques may be used. For example, in some embodiments, a portion of flow from the supply line may be redirected to the reservoir to dissolve and/or dilute the concentrate or powder mixture, and then subsequently re-introduced into the remaining flow of the supply line.

The present invention also provides a method of reducing formation of or eliminating aerosol droplets generated during surgical irrigation procedures and dental treatment procedures. The method comprises providing an aqueous irrigation composition of the present invention and introducing the composition into a surgical area, such as a dental treatment area, at ambient pressures or in a pressurized form, wherein the aerosolization is reduced compared to aerosolization generated by use of the aqueous composition which does not contain a polymer.

The method reduces or eliminates aerosol particle formation during an irrigation procedure (*e.g*., a dental procedure). In various examples, the method comprises irrigating the oral cavity of an individual with an aqueous composition comprising a polymer. The aerosolization produced from and during the dental procedure is less than the aerosolization with an aqueous medium in the absence of the composition according to the invention. In an embodiment, the composition is an irrigation composition with low aerosolization. The irrigation composition is suitable for irrigating biological cavities/surfaces or for use with dental or surgical instrumentation. The present compositions and methods may be used together with the use of ultrasonic, rotary or kinetic agitators used in treatment areas, such as dental treatment area. The present composition and methods may be used to reduce aerosolization during cleaning (*e.g*., sterilization) of surgical or other instruments or surfaces relevant in a clinical setting, such as, for example, using an ultrasonic bath where the bath contains the composition.

The present compositions may be provided as aqueous compositions ready-to-use or may be provided as concentrated liquids, or as powders. The concentrated liquids or powders may be prepared into usable compositions at the point of use or any point or time before that.

In various examples, the composition is supplied in various forms (*e.g*., as concentrate or in final dilution) to the dentist or dental hygienist. Examples include where the final concentration of polyethylene oxide can be filled directly in the liquid tanks of the scaler, or a concentrate of polyethylene oxide for dilution either prior to use in scaler units with liquid tank or for automatic dilution in the feed unit of the dental chair unit (similar to hitherto employed liquid feed disinfection concentrates). Optionally, an in-can preservative can be added against microbial contamination (*e.g*., standard FDA approved preservatives). For example, a concentrated composition has a concentration 5 - 100x more concentrated than 0.01 wt.% (100 ppm) to 0.1 wt.% (1000 ppm).

The concentrated liquid or powder mixture is preferably mixed with water such that the resulting aqueous composition comprises 100 ppm to 2000 ppm, preferably 100 ppm to 1000 ppm, more preferably 200 to 500 ppm, and most preferably 200 to 400 ppm of the at least one hydroxyalkyl cellulose and/or poly(ethylene oxide) with a molecular weight of 600,000 to 12 million g/mol, preferably 8 million g/mol.

The compositions of the present invention are preferably provided in the form of single-use containers which comprise a powder, or preferably a concentrate, in the amount necessary to prepare a certain amount of the aqueous composition. Preferably the single-use container comprises 1 to 4 g, more preferably 2 to 3 g, and most preferably about 2 g of the powder or concentrate and is adapted to be diluted with water to about 1 liter.

The compositions may be used in a variety of treatment procedures where irrigation liquids are used. For example, the compositions and methods may be performed on an individual who is undergoing a surgical or dental procedure. Examples of dental procedures include, but are not limited to, ultrasonic, rotary of kinetic cleaning, particle abrasion polishing, cavity preparation, extension of fissures, root canal preparation, adjustment of occlusion during restorative work, implantation, medical procedures (*e.g*., ultrasonic debridement of wounds, water-cooled drilling procedures of bone, irrigation, and the like), events requiring air or water spray irrigation, and the like. The present compositions may be incorporated into currently existing irrigation equipment and waterlines, such as those found in a dental examination/treatment room. In various examples, the compositions may be used in an ultrasonic bath. In various other examples, the composition may be used as a coolant.

All of above aspects used in human medicine may be similarly applied to veterinary use. For example, any of the above aspects may be applied to any surgical procedure procedural used in veterinary care that generates an aerosol.

In an aspect, the present disclosure provides kits. The kits may comprise a composition of the present invention or it may comprise a composition of the present disclosure dispensed into ready-to-use containers for surgical or dental procedures.

A kit may comprise separate containers for the composition according to the invention and the appropriate amount of an aqueous medium, such as water, to prepare a composition for use in rinsing a treatment area. The water or the container containing the polymer may comprise additives, or the additive many be provided separately. For example, a kit may comprise a polymer, sterile water, and optionally, additives. The polymer may be provided in any form, such as concentrated liquid or powder. The kit may further comprise disposable items, such as, for example, hoses, fittings, and other items that may be used to deliver a composition of the present disclosure. The kit may further comprise instructions for preparing and/or using the composition. The kit may further comprise one or more article for interfacing a vessel (*e.g*., a bottle or other container containing the composition) with an irrigation system. The kit may comprise combinations of various disposable items.

A number of embodiments can be constructed in accordance with the principles herein. The following exemplary embodiments are provided to demonstrate these principles, and to demonstrate that other examples listed below achieve results in accordance with the principles herein.

### EXAMPLE 1

The following concentrate formulation was prepared by homogeneously mixing the compounds listed in the table below. Glycerol and PEG 400 were filled in a mixing container, then PEG 8 Mio was added while stirring. Stirring was continued for 0.5 to 1.5 hours until a homogeneous suspension was obtained. The composition was filled into single-dose containers, each containing 2 g of the composition.

| **Component** | **Amount [%]** |
|---|---|
| Glycerol | 64.50 % |
| PEG 400¹⁾ | 24.50 % |
| Aerosil 200³⁾ | 1.00 % |
| PEO 8 Mio²⁾ | 10.00 % |

| | |
|---|---|
| ¹⁾ polyethylene oxide) with an average molecular weight of 400 g/mol ²⁾ polyethylene oxide with the average molar mass of 8 million g/mol (Polyox WSR 308, AMERCHOL), sieved, 200 µm ³⁾ fumed silica, BET surface area 175-225 m²/g, primary particle size 12 nm | |

For use, about 300 to 500 ml of water were filled into a one liter bottle. The suspension from one single-dose container (2 g) was added and the bottle was shaken vigorously for 30 seconds. The remaining water (to 1000 ml) was added and the bottle was allowed to stand for 10 minutes. The bottle was shaken again for another 5 s and then used in an ultrasonic scaler.

### EXAMPLE 2

The following concentrate formulation was prepared analogously to Example 1 and filled into single-dose containers, each containing 2 g of the composition.

| **Component** | **Amount [%]** |
|---|---|
| Glycerol | 65.00 % |
| PEG 400¹⁾ | 25.00 % |
| PEO 8 Mio²⁾ | 10.00 % |

| | |
|---|---|
| ¹⁾ polyethylene oxide) with an average molecular weight of 400 g/mol ²⁾ polyethylene oxide with the average molar weight of 8 million g/mol (Polyox WSR 308, AMERCHOL), sieved, 200 µm | |

The suspension from one single-dose container (2 g) was mixed with water as described in Example 1 and then used in an ultrasonic scaler.

### EXAMPLES 3 to 26

The following concentrates were prepared analogously to Example 1:

| **No.** | **Carrier** | **Polymer** | **Conc. [wt.%]** | **Aerosil 200³⁾ [wt.%]** | **Performance⁵⁾** |
|---|---|---|---|---|---|
| 3 | NaHCO₃ | PEO 8 Mio²⁾ | 9.00 | - | n.d. |
| 4 | NaHCO₃ | PEO 8 Mio²⁾ | 20.00 | - | n.d. |
| 5 | NaHCO₃/Na₂CO₃ | PEO 8 Mio²⁾ | 50.00 | - | n.d. |
| 6 | NaHCO₃ | 2-Hydroxyethyl cellulose⁴⁾ | 20.00 | - | n.d. |
| 7 | NaHCO₃ | 2-Hydroxyethyl cellulose⁴⁾ | 50.00 | - | n.d. |
| 8 | Glycerol | PEO 8 Mio²⁾ | 10.00 | - | n.d. |
| 9 | Glycerol | PEO 8 Mio²⁾ | 15.00 | - | 5 |
| 10 | Glycerol | PEO 8 Mio²⁾ | 20.00 | - | 5 |
| 11 | Glycerol | PEO 8 Mio²⁾ | 25.00 | - | 5 |
| 12 | PEG 400¹⁾ | PEO 8 Mio²⁾ | 6.00 | 5 | n.d. |
| 13 | Glycerol | PEO 8 Mio²⁾ | 6.00 | 5 | 5 |
| 14 | Glycerol | PEO 8 Mio²⁾ | 20.00 | 3 | 5 |
| 15 | Glycerol | PEO 8 Mio²⁾ | 20.00 | 3 | n.d. |
| 16 | Glycerol | PEO 8 Mio²⁾ | 10.00 | 5 | n.d. |
| 17 | Glycerol | PEO 8 Mio²⁾ | 10.00 | 5 | n.d. |
| 18 | Glycerol/PEG400¹⁾ (80/10) | PEO 8 Mio²⁾ | 10.00 | - | 2 |
| 19 | Glycerol/PEG400¹⁾ (85/5) | PEO 8 Mio²⁾ | 10.00 | - | 3 |
| 20 | Glycerol/PEG400¹⁾ (70/20) | PEO 8 Mio²⁾ | 10.00 | - | 4 |
| 21 | Glycerol/PEG400¹⁾ (60/30) | PEO 8 Mio²⁾ | 10.00 | - | 4 |
| 22 | Glycerol/PEG400¹⁾ (50/40) | PEO 8 Mio²⁾ | 10.00 | - | 2 |
| 23 | Glycerol/PEG400¹⁾ (40/50) | PEO 8 Mio²⁾ | 10.00 | - | 2 |
| 24 | Glycerol/PEG400¹⁾ (65/25) | PEO 8 Mio²⁾ | 10.00 | - | 5 |
| 25 | Glycerol/PEG400¹⁾ (55/35) | PEO 8 Mio²⁾ | 10.00 | - | 5 |
| 26 | Glycerol/PEG400¹⁾ (67/23) | PEO 8 Mio²⁾ | 10.00 | - | n.d. |

| | | | | | |
|---|---|---|---|---|---|
| ¹⁾ polyethylene oxide) with an average molecular weight of 400 g/mol ²⁾ polyethylene oxide with the average molar weight of 8 million g/mol (Polyox WSR 308, AMERCHOL), sieved, 200 µm ³⁾ fumed silica, BET surface area 175 - 225 m²/g, primary particle size 12 nm ⁴⁾ average molecular weight 380,000 g/mol ⁵⁾ Grade 5 = excellent, Grade 1 = no effect, n.d. = not determined | | | | | |

To determine their stability, the compositions were stored at 50°C for 11 weeks and then their effect on aerosol formation was tested. The test was carried out using an EMS Piezon 250 ultrasonic scaler with an external water tank. For the test, 500 ml of water was added to a 1000 ml container. The composition to be tested was added in such an amount that the concentration of PEO was 0.2 g/1000 ml. This mixture was shaken vigorously for 30 seconds. A further 500 ml of water was then added, and the solution was left to rest for 10 minutes. This solution was shaken again for 5 seconds and then added to the water tank of the scaler.

The scaler's irrigation power was set to a middle level (position 5 of 9) and the scaler was then started. Before the test, it was allowed to run for one minute to remove any remaining water from the hoses. The jet of cooling water emitted from the tip of the handpiece was then observed for one minute and jet characteristics and splash formation were evaluated using the following criteria:

| **Rating** | **Assessment** | **Description** |
|---|---|---|
| 5 | excellent | continuous water jet without aerosol generation or splashes |
| 4 | very good | continuous water jet with very few splashes (1-2 splashes/min) |
| 3 | good | continuous water jet with few splashes (3-4 splashes/min) |
| 2 | acceptable | continuous water jet with reduced aerosol generation |
| 1 | very poor | aerosol generation as with pure water, no effect |

The results are shown in the above table. It is evident that the compositions according to the invention are storage stable, i.e. they maintain their property to suppress aerosol formation.

## Claims

1. A composition comprising at least one liquid or solid carrier and at least one polymer, **characterized in that** the at least one polymer comprises at least hydroxyalkyl cellulose polymer, at least one polyethylene oxide) polymer or a mixture thereof and that the at least one polymer is not soluble in the liquid carrier.

2. The composition of claim 1, comprising at least one poly(ethylene oxide) with an average molecular weight of 600,000 to 12 million g/mol, preferably 3 million to 8 million g/mol and/or at least one hydroxypropyl cellulose or hydroxyethyl cellulose with an average molecular weight of 300,000 to 450,000 g/mol, preferably 370,000 to 380,000 g/mol.

3. The composition of claim 1 or 2, comprising a liquid carrier selected from alcohols, preferably polyols, fatty acids, ethers, and DMSO, or a solid carrier selected from sugars, preferably monosaccharides, disaccharides, or polysaccharides, sugar alcohols, preferably xylitol or sorbitol, citric acid, tartaric acid, solid low molecular weight polyethylene glycols, preferably PEG 1000, gelatin, polyacrylates, polyvinyl alcohols, gum arabic, saccharin, cyclamate, salts, preferably (NH₄)₂SO₄, K₂SO₄, Na₂SO₄, MgSO₄, NaCl, NaBr, MgCl₂, Na₂HPO₄, CaHPO₄, Na₂CO₃ and NaHCO₃.

4. The composition of claim 3, comprising at least one polyethylene oxide) with an average molecular weight of 200 to 600 g/mol, preferably 400 g/mol, glycerol or a mixture thereof.

5. The composition of any one of claims 1 to 4, comprising
- 50 to 99 wt.%, preferably 69 to 97 wt.% and more preferably 90 wt.% of the liquid carrier; and
- 1 to 50 wt.%, preferably 3 to 31 wt.% and more preferably 10 wt.% of at least one hydroxyalkyl cellulose or preferably at least one polyethylene oxide) having a molecular weight of 600,000 to 12 million g/mol, or comprising
- 1 to 80 wt.%, preferably 9 to 50 wt.%, of at least one hydroxyalkyl cellulose or preferably at least one poly(ethylene oxide) with a molecular weight of 600,000 to 12 million g/mol, and
- 20 to 99 wt.%, preferably 50 to 91 wt.%, of a solid carrier, preferably NaHCOs,
in each case based on the total mass of the composition.

6. The composition of claim 4, comprising
- 36 to 71 wt.%, preferably 50 to 70 wt.%, and more preferably 65 wt.% of glycerol;
- 14 to 28 wt.%, preferably 19 to 27 wt.% and more preferably 25 wt.% of at least one polyethylene oxide) having a molecular weight of 200 to 600 g/mol, and
- 1 to 50 wt. %, preferably 3 to 31 wt.% and more preferably 10 wt.% of at least one poly(ethylene oxide) having a molecular weight of 600,000 to 12 million g/mol,
or comprising
- 36 to 71 wt.%, preferably 40 to 70 wt.% and more preferably 65 wt.% glycerol;
- 14 to 35 wt.%, preferably 19 to 35 wt.% and more preferably 25 wt.% of at least one at least one polyethylene oxide) having a molecular weight of 200 to 600 g/mol, and
- 1 to 29 wt.%, preferably 3 to 25 wt.% and more preferably 10 wt.% of at least one poly(ethylene oxide) having a molecular weight of 600,000 to 12 million g/mol,
in each case based on the total mass of the composition.

7. The composition of any one of claims 4 to 6, wherein the at least one polymer is a poly(ethylene oxide) polymer with an average molecular weight of 600,000 to 12 million g/mol, preferably 3 to 8 million g/mol, and the at least one liquid or solid carrier is a liquid carrier selected from glycerol, poly(ethylene oxide) with an average molecular weight of 400 g/mol, a mixture thereof, and preferably is glycerol.

8. The composition of any one of claims 1 to 7, further comprising one or more additives, wherein the one or more additives are preferably chosen from thickeners, antioxidants or antioxidant-synergists, chelating agents, flavorings, colorings, antimicrobials, preservatives, biofilm removal agents, calculus or plaque staining dyes, wetting agents/detergents, and combinations thereof.

9. The composition according to any one of claims 1 to 8, comprising less than 1.0 wt.% of water and/or 0 to 5 wt.% of one or more additives, based on the total mass of the composition.

10. The composition according to claim 9, consisting essentially of the said compounds.

11. Aqueous composition comprising the composition of any one of claims 1 to 10 and water, preferably in such amounts that the aqueous composition comprises 100 ppm to 2000 ppm, preferably 100 to 1000 ppm, more preferably 200 to 500 ppm, and most preferably 200 to 400 ppm of the at least one poly(ethylene oxide) with a molecular weight of 600,000 to 12 million g/mol.

12. A system for supplying an aqueous composition to a dental instrument, comprising:
- a reservoir containing the composition of any one of claims 1 to 11,
- a supply line in fluid communication with the reservoir, the supply line configured to supply the composition of the reservoir to a dental instrument as an aqueous composition.

13. A method of reducing or eliminating formation of aerosol droplets during a non-therapeutic dental irrigation procedure comprising irrigating a treatment area of an individual with the aqueous composition according to claim 11.

14. A kit comprising:
(a) a first container containing the composition of any one of claims 1 to 10;
(b) a second container containing an aqueous medium in amount sufficient for mixing with (a) to prepare a composition comprising 100 ppm to 2000 ppm of the at least one polyethylene oxide) with a molecular weight of 600,000 to 12 million g/mol, and optionally
(c) one or more disposable items chosen from hoses, fittings, bottles, articles for interfacing a vessel containing the composition with an irrigation system, and combinations thereof.

15. The composition according to any one of claims 1 to 11 for reducing or eliminating formation of aerosol droplets during a therapeutic medical or dental irrigation procedure.

16. Use of a composition according to any one of claims 1 to 11 for reducing or eliminating formation of aerosol droplets during a non-therapeutic medical or dental irrigation procedure.

17. Single use container comprising the composition of any one of claims 1 to 10.
